# EUROPEAN PATENT APPLICATION

(11) **EP 4 089 415 A1**
(43) Date of publication of application: **16.11.2022**
(21) Application number: 21382448.5
(22) Date of filing: 14.05.2021
(51) Int. Cl.: G01N 33/574

(54) **IN VITRO METHOD FOR THE DIAGNOSIS OR PROGNOSIS OF BREAST CANCER**

(71) Applicant: Servizo Galego de Saúde (SERGAS), 15703 Santiago de Composteia (ES); Fundación Instituto de Investigación Sanitaria de Santiago de Compostela, 15706 Santiago de Compostela, A Coruña (ES); Universidade de Santiago de Compostela, 15782 Santiago de Compostela, A Coruña (ES); Roche Farma, S.A.U., 28042 Madrid (ES)
(72) Inventor: COSTA NOGUEIRA, Clotilde, 15706 Santiago de Compostela (A Coruña) (ES); YÁÑEZ GÓMEZ, Celso, 15706 Santiago de Compostela (A Coruña) (ES); BRAVO LÓPEZ, Susana, 15706 Santiago de Compostela (A Coruña) (ES); LÓPEZ LÓPEZ, Rafael, 15706 Santiago de Compostela (A Coruña) (ES); GÓMEZ TATO, Antonio, 15782 Santiago de Compostela (A Coruña) (ES)
(74) Representative: Hoffmann Eitle

(57) **Abstract**

The present invention refers to an *in vitro* method for the diagnosis and/or prognosis of breast cancer. The method of the invention is carried out by determining the concentration level of proteins in erythrocytes or red blood cells (RBCs) which are present in- or isolated from patient blood samples.

## Description

### FIELD OF THE INVENTION

The present invention refers to the medial field. Particularly, the present invention refers to an *in vitro* method for the diagnosis and/or prognosis of breast cancer. The method of the invention is carried out by determining the concentration level of proteins in erythrocytes or red blood cells (RBCs) comprised in patient blood samples.

### STATE OF THE ART

Metastasis is an evolutionary process, resulting in significant molecular differences between metastasis and the primary tumour. An alternative to a classic biopsy is the Liquid Biopsy analysis which refers to the study of tumour material for example circulating tumour DNA (ctDNA), CTCs (Circulating Tumour Cells), exosomes, etc. from the blood. The Liquid Biopsy offers multiple advantages, since it is a non-invasive method that allows easily obtaining multiple samples at different time points of the disease. The release of DNA into the circulation occurs both from the primary tumour, the CTCs, and the metastases in distal locations. Therefore, CTCs can provide real-time information on tumour status in a systemic manner, facilitating the study of mechanisms that modify response to therapy and tumour dissemination.

The presence of CTCs in the blood of cancer patients is low (on average, 1 CTC per 10⁷ blood cells) and there are still many challenges unreached regarding an efficient isolation and enumeration of CTCs. In fact, there is only a system with the Food and Drug Administration approval for CTCs enumeration: CellSearch^{®} technology.

In recent years there are evidences that point to red blood cells (RBCs) as markers of health and disease. Thus, RBCs abnormalities in Type 2 Diabetes and Alzheimer's Disease are relevant events that involve the impairment of RBCs morphology, deformability, and function accompanied by oxidative stress-related events in both diseases, leading to vascular dysfunctions. Similarly, RBCs alteration takes place in various subtypes of Multiple Sclerosis, rheumatoid arthritis disease and/or its concomitant factors or atherosclerotic disease progression due to the crosstalk between RBCs with immune cells. In addition, enhanced eryptosis (apoptosis-like cell death of RBCs) shortens the lifespan of circulating RBCs and confers a procoagulant phenotype. This phenomenon has been tangibly implicated in the pathogenesis of anaemia, deranged microcirculation and increased pro-thrombotic risk associated with a multitude of clinical conditions. Besides, enhanced eryptosis has been described in lung cancer and in myelodysplastic syndrome and in mice with intestinal tumours because of adenomatous polyposis coli deficiency. It is noteworthy an article published in 2010 [Assmus, B. et al. Red Blood Cell Contamination of the Final Cell Product Impairs the Efficacy of Autologous Bone Marrow Mononuclear Cell Therapy. J. Am. Coll. Cardiol. (2010). doi:10.10161j.jacc.2009.10.059] in which is described that the contamination of the isolated Bone Marrow Mononuclear cells with RBCs is a significant independent predictor of reduce recovery in cardiovascular regenerative therapies in patients with acute myocardial infarction. Moreover, they correlated the highest dose of RBCs contamination with the migratory capacity of Bone Marrow cells, revealing an active role of RBCs mediated by mitochondria membrane potential. Besides, in mostly affecting women with localized Breast Cancer (BC) of age > 70, cardiovascular disease was the leading cause of death and a major contributor to total mortality. Furthermore, a recent study in children with neuroblastoma [Morandi, F. et al. Altered erythropoiesis and decreased number of erythrocytes in children with neuroblastoma. Oncotarget 8, 53194-53209 (2017*)]* described altered erythropoiesis, suggesting that primary tumour cells produce effects at distance and that the observed impairment could be mediated by extracellular vesicles released by the tumour cells. Other recent proposed functions for RBCs are clearing bacteria from the bloodstream and epimmunity or single cell defense against infectious diseases. These theories abrogate for RBCs as participants in defense mechanisms within the body and this fact could explain the relation among cancer and sepsis. In another vein, it is described that CTCs adhesion to microvessel walls depends on blood viscosity. Thus, RBC aggregation enables the CTCs stably roll along the vessel wall at a low flow rate. Furthermore, the wall shear stress significantly contributes to tumour cell adhesion by activating or inactivating cell adhesion molecules. From a physiological point of view, the binding affinity of the cell adhesion molecules could be enhanced or weakened by the variation of the shear stress in the wall. Thus, the tumour and endothelial cells are activated, giving rise to an increase of adhesion bounds if the shear stress in the wall is positive.

Regarding RBCs interaction with tumour cells, there are only two works related. Thus, Helwa and colleagues reported that tumour cells interact with RBCs via galectin-4 *[*Helwa, R., Heller, A., Knappskog, S. & Bauer, A. S. Tumor cells interact with red blood cells via galectin-4 - a short report. Cell. Oncol. 40, 401-409 (2017*)]* and it is known that RBCs membrane protein composition is altered in advanced non-small cell lung cancer [Hernández-Hernández, A. et al. Alterations in erythrocyte membrane protein composition in advanced non-small cell lung cancer. Blood Cells, Mol. Dis. (2006). doi:10.1016/j.bcmd.200602.002] although, in this work, only four proteins were analysed by western blot. On the other hand, the following study was carried out in women with ovarian cancer, wherein the quantitative protein evaluation showed a statistically significant decrease in spectrin and a significant increase in 4.1 protein *[*British journal of cancer (1998) 78(4), 466-471]. Other study in breast cancer patients concluded that quantitative protein evaluation showed an increase in band 4.1 protein content with no changes in the level of constitutive PKC responsible for the phosphorylation of this protein and its affinity to glycophorine C. In parallel a greater increase of PKCα translocation after PMA treatment compared to controls was observed. Possible oxidative damage of RBCs membranes indicated by an increase in malonyldialdehyde level and decrease in SH-group content as well as by an increase in the w/s ratio was documented. From the results it is concluded that primary breast cancer seems to affect the membranes of mature RBCs *[*Blood Cells, Molecules, and Diseases (2002) 29(2) Sept/Oct: 225-235].

RBCs comprise 67.6-83.3% of the total host cells in the human body and every day, 0.2 trillion RBCs are generated and removed from the bloodstream, implying that a significant amount of the daily energy expenditure is dedicated to RBCs homeostasis. This suggests a central role for RBCs in human physiology. Traditionally, RBCs were considered merely oxygen transporters. However, a long list of critical functions has already been attributed and recent studies have demonstrated an unforeseen complexity and novel roles for RBCs in the systemic metabolic homeostasis in health and disease. Thus, RBCs are now considered cells with systemic influence and a dynamic relationship with its environment. RBCs have a life-span of 120 days and therefore may accumulate modifications in their proteins that represent the long-term status of other parts of the body. Consequently, they serve as markers of specific diseases and their evolution. It has been speculated that comparative proteomic analysis of RBCs in health and disease would provide information about the function of genes and environmental effects, as well as on interactions between them. This would give potential RBCs markers and risk factors for different diseases, including cancers *[*Pasini, E. M., Lutz, H. U., Mann, M. & Thomas, A. W. Red blood cell (RBC) membrane proteomics--Part II: Comparative proteomics and RBC patho-physiology. J. Proteomics (2010). doi:10.1016/j.jprot.2009.07.004]. RBCs circulate in plasma together with a diversity of cells, therefore, many alterations within and on RBCs may result from contact with plasma proteins, with drugs added to plasma, with substances released from activated cells and likewise with nucleated cells as CTCs. Furthermore, RBCs release protein factors with the capacity to sustain malignant cell growth, proliferation and survival *in vivo.*

Thus, are capable to regulate biological processes of neighbouring cells, becoming as a novel regulatory cell. Although, RBCs were historically considered inert to regulatory signals from other cells, they are well equipped with the machinery required for intercellular communication. In fact, marked changes of RBCs deformability and aggregation have been shown to be mediated by activation of different intracellular signalling pathway. In another vein, after stimulation, almost all cells could work releasing extracellular vesicles (EVs). Most of them in human blood originate from platelets, but EVs can be generated from white blood cells (WBC), RBCs, the endothelium, smooth muscle cells and cancer cells. Platelet-derived EVs enhance chemotaxis in response to stromal cell-derived factor 1, leading to cancer progression or metastasis. Consistently, the concentration of circulating platelet derived EVs differs by cancer stage. However, little is known regarding RBCs derived EVs performance.

In cancer patients are usually found altered states as coagulation, cachexia or sepsis that could result from alteration of RBCs physiology. Thus, RBCs could reflect tumour state. Several reports have reinforced the concept of a connection between activated clotting and angiogenesis in human cancer, however, the precise coagulation mechanism that increases the success of tumour cells remains to be established *[*Palumbo, J. S. & Degen, J. L. Fibrinogen and tumor cell metastasis. Haemostasis (2001*)].* In addition, Young Bang and colleagues hypothesized that the EVs of cancer cells cause coagulopathy resulting in a cancer-related stroke by directly stimulating factor X *[*Bang, O. Y. et al. Cancer cell-derived extracellular vesicles are associated with coagulopathy causing ischemic stroke via tissue factor-independent way: The OASIS-CANCER study. PLoS One (2016). doi:10.1371/journal.pone.0159170]*.* It has been reported an anaemia-inducing substance (AIS) secreted by the malignant cells that made the RBCs osmotically fragile and decreased their deformability in the plasma of the patient with terminal cancer *[*Ishiko, O. et al. Anemia-inducing substance (AIS) in advanced cancer: inhibitory effect of AIS on the function of erythrocytes and immunocompetent cells. Japanese J. Cancer Res. (1987*)] and [*Honda, K. et al. Anemia-inducing Substance from Plasma of Patients with Advanced Malignant Neoplasms. Cancer Res. 55, 3623 LP-3628 (1995*)*]. This event had been related with cancer cachexia, in which the main cause of death is due to respiratory failure. On the other side, cancer patients usually have compromised immune status. Therefore, the disease itself or the aggressive treatments against cancer can give rise to infections development that is usually found in the tumour environment. Another important factor is that cancer patients often receive blood transfusions, which has been shown to be likely to pose an additional risk of infectious complications. Thus, there are a significant percentage of cancer deaths related to sepsis. Since RBCs are responsible for clearing bacteria from the bloodstream, RBCs alterations will affect their appropriate functions, leading to increase sepsis risk. Other important hint to consider is the analytical parameter Red cell distribution width (RDW) which is a measure of the variation in the volume of RBCs that has been proposed as a risk factor for death. Thus, a meta-analysis has shown that RDW may be a potential prognostic marker in cancer patients, and high RDW may also be associated with poor outcomes. However, there is some controversy, since some authors support the relationship between RDW and cancer is a reflection of the effect that inflammation and oxidative stress causes on RBCs and that, in fact, are also cancer risk factors.

Each step of tumour development, from initiation through metastatic spread, is promoted by communication between tumour and immune cells via the secretion of cytokines, growth factors and proteases that remodel the tumour microenvironment. Current knowledge adds epigenetics and miRNAs as important regulators of metastatic events; however, little is known regarding RBCs contribution.

### DESCRIPTION OF THE INVENTION

### Brief description of the invention

The present invention refers to an *in vitro* method for the diagnosis and/or prognosis of breast cancer. The method of the invention is carried out by determining the concentration level of proteins in RBCs comprised in patient blood samples. Since the RBCs are comprised in patient blood samples, the method of the invention can be carried out by either working directly with blood samples (since RBCs are the most abundant cell in the blood, accounting for about 40 to 45 percent of its volume and around 95% of the cellular components of blood). As an alternative, the RBCs could be isolated departing from blood samples obtained from the patient.

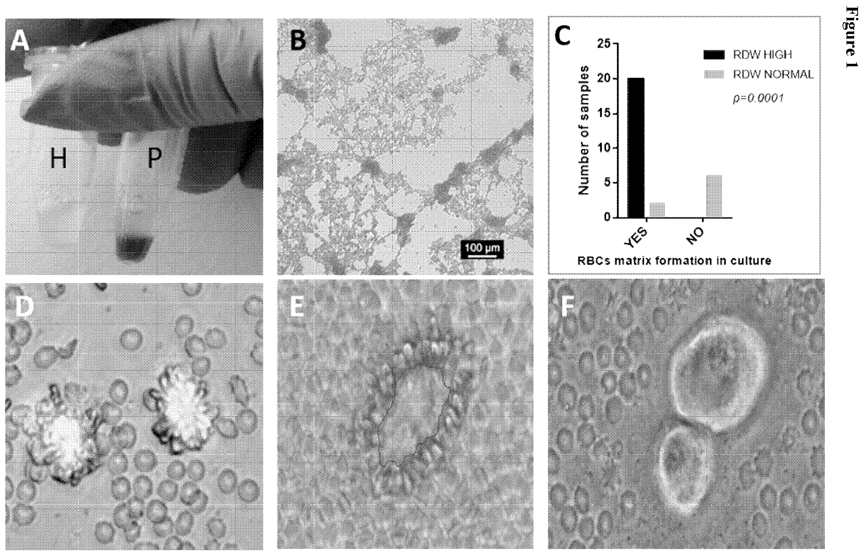

Therefore, the present invention is based on the fact that RBCs of cancer patients behave differently than RBCs of healthy subjects (see **Figure 1****).** The RBCs derived from cancer patients tend to form rosettes and also adhere to the CTCs and to cancer cell lines *in vitro.*

To understand what may be causing the differences in the behaviour of the RBCs, the inventors of the present invention compared the concentration profile of proteins derived from RBCs in patients suffering from breast cancer with the concentration profile of the proteins derived from RBCs of healthy subjects (or non-oncologic subjects).

The inventors have found a group of proteins differentially expressed/regulated in breast cancer patients. Particularly, such as it is indicated in **Table 1,** said proteins were differentially expressed in metastatic breast cancer patients (MBC) *vs* healthy controls (CT), in MBC *vs* non-metastatic breast cancer patients (NMBC), and in NMBC *vs* CT.

**Table 1**

| **PROTEIN NAME** | **t-test (p value)** | | | **Fold Change** | | |
|---|---|---|---|---|---|---|
| | **MBC vs CT** | **NMBC vs CT** | **MBC vs NMBC** | **MBC vs CT** | **NMBC vs CT** | **MBC vs NMBC** |
| GNAQ | 0,0000 | 0,4571 | 0,0010 | 2,2313 | 1,1579 | 1,9271 |
| PSA7 | 0,0014 | 0,0233 | 0,4746 | 1,2110 | 1,1557 | 1,0478 |
| HBD | 0,0031 | 0,0147 | 0,7487 | 1,5373 | 1,4611 | 1,0522 |
| A1AG1 | 0,0072 | 0,1258 | 0,3643 | 1,4369 | 1,2527 | 1,1470 |
| CD59 | 0,0078 | 0,1224 | 0,3538 | 0,6880 | 0,7936 | 0,8669 |
| LAMP2 | 0,0100 | 0,0299 | 0,7678 | 1,7355 | 1,6222 | 1,0698 |
| MIF | 0,0114 | 0,5180 | 0,0929 | 1,3773 | 1,0902 | 1,2633 |
| PLSL | 0,0127 | 0,8883 | 0,0168 | 1,4201 | 0,9793 | 1,4501 |
| TPM3 | 0,0130 | 0,8917 | 0,0340 | 0,7676 | 0,9846 | 0,7796 |
| FN3K | 0,0138 | 0,1129 | 0,4793 | 0,7400 | 0,8133 | 0,9099 |
| 6PGL | 0,0147 | 0,4219 | 0,1493 | 1,2800 | 1,0902 | 1,1740 |
| CATA | 0,0154 | 0,3720 | 0,1799 | 0,8518 | 0,9391 | 0,9071 |
| ADDA | 0,0174 | 0,0002 | 0,1328 | 0,8039 | 0,6905 | 1,1641 |
| K22E | 0,0184 | 0,3997 | 0,1841 | 0,7378 | 0,8908 | 0,8282 |
| PNPH | 0,0217 | 0,0676 | 0,7562 | 1,1511 | 1,1273 | 1,0211 |
| PARVB | 0,0246 | 0,5673 | 0,1368 | 0,6814 | 0,9013 | 0,7560 |
| IGLC3 | 0,0250 | 0,8757 | 0,0618 | 1,3663 | 1,0235 | 1,3350 |
| S10A8 | 0,0258 | 0,0880 | 0,7124 | 1,2920 | 1,2333 | 1,0476 |
| LKHA4 | 0,0274 | 0,0852 | 0,7001 | 1,8439 | 1,6403 | 1,1241 |
| A1AT | 0,0283 | 0,8203 | 0,0771 | 1,1864 | 1,0190 | 1,1643 |
| F10A1 | 0,0297 | 0,3031 | 0,3257 | 0,8668 | 0,9304 | 0,9317 |
| MMP9 | 0,0311 | 0,1597 | 0,5523 | 1,4136 | 1,2728 | 1,1106 |
| PSMD9 | 0,0324 | 0,2313 | 0,4307 | 1,4899 | 1,2686 | 1,1744 |
| UBP5 | 0,0331 | 0,0072 | 0,4979 | 0,7886 | 0,7256 | 1,0870 |
| CO3 | 0,0331 | 0,4971 | 0,2016 | 0,7868 | 0,9218 | 0,8535 |
| CPNS1 | 0,0337 | 0,3027 | 0,3522 | 0,7453 | 0,8588 | 0,8679 |
| CHMP6 | 0,0341 | 0,3537 | 0,3018 | 1,3062 | 1,1320 | 1,1539 |
| TKT | 0,0341 | 0,0682 | 0,8801 | 0,8855 | 0,8940 | 0,9905 |
| RHAG | 0,0347 | 0,3401 | 0,3246 | 1,2979 | 1,1348 | 1,1437 |
| SORCN | 0,0381 | 0,2875 | 0,3934 | 1,2232 | 1,1166 | 1,0955 |
| PGK1 | 0,0408 | 0,0676 | 0,9371 | 0,8801 | 0,8849 | 0,9946 |
| GLPC | 0,0411 | 0,2189 | 0,5520 | 1,3961 | 1,2500 | 1,1169 |
| HS90B | 0,0414 | 0,1349 | 0,6750 | 0,5346 | 0,6150 | 0,8693 |
| H4 | 0,0458 | 0,8621 | 0,0502 | 1,3048 | 0,9758 | 1,3371 |
| IDHP | 0,0480 | 0,7981 | 0,1226 | 1,4933 | 1,0569 | 1,4130 |
| ALBU | 0,0483 | 0,0460 | 0,8793 | 0,8603 | 0,8494 | 1,0128 |
| SODM | 0,0494 | 0,0347 | 0,7910 | 1,5335 | 1,6334 | 0,9388 |
| ARF3 | 0,0597 | 0,1157 | 0,0014 | 0,8280 | 1,1841 | 0,6993 |
| RD23A | 0,0670 | 0,0045 | 0,2654 | 0,7606 | 0,6332 | 1,2012 |
| ZN493 | 0,1493 | 0,2695 | 0,0184 | 0,7678 | 1,2421 | 0,6182 |
| CAH3 | 0,1563 | 0,1959 | 0,0119 | 0,8671 | 1,1497 | 0,7542 |
| TALDO | 0,1829 | 0,0385 | 0,4166 | 0,9069 | 0,8491 | 1,0681 |
| RINI | 0,1887 | 0,0008 | 0,0352 | 0,8726 | 0,6844 | 1,2749 |
| PEBP1 | 0,1994 | 0,0372 | 0,3788 | 0,8417 | 0,7379 | 1,1406 |
| ALDOA | 0,2045 | 0,0553 | 0,0029 | 1,1268 | 0,8235 | 1,3683 |
| AP1B1 | 0,2127 | 0,0122 | 0,1729 | 1,2200 | 1,5690 | 0,7776 |
| NTF2 | 0,2131 | 0,2693 | 0,0275 | 1,2485 | 0,8108 | 1,5398 |
| AACT | 0,2562 | 0,0137 | 0,1645 | 0,8598 | 0,6993 | 1,2294 |
| GAPR1 | 0,2926 | 0,0092 | 0,1094 | 0,7424 | 0,4458 | 1,6652 |
| ACLY | 0,2927 | 0,0263 | 0,2236 | 0,8851 | 0,7571 | 1,1692 |
| IGHG2 | 0,3039 | 0,0487 | 0,3231 | 0,7720 | 0,5841 | 1,3216 |
| TRFE | 0,3253 | 0,0193 | 0,1631 | 0,8449 | 0,6481 | 1,3036 |
| PA1B2 | 0,3481 | 0,0119 | 0,1080 | 1,0680 | 1,2103 | 0,8824 |
| 1433Z | 0,3741 | 0,0056 | 0,0571 | 0,8948 | 0,6867 | 1,3030 |
| STOM | 0,3765 | 0,1815 | 0,0372 | 0,9488 | 1,0891 | 0,8712 |
| RMD5B | 0,4407 | 0,0674 | 0,0168 | 0,8664 | 1,4455 | 0,5994 |
| JAM1 | 0,4684 | 0,0419 | 0,1845 | 1,1362 | 1,4706 | 0,7726 |
| KAD1 | 0,4850 | 0,0104 | 0,0616 | 1,0378 | 1,1589 | 0,8955 |
| ACPH | 0,6547 | 0,0168 | 0,0067 | 1,0344 | 0,8224 | 1,2578 |
| CISY | 0,8220 | 0,0307 | 0,0546 | 1,0483 | 1,6287 | 0,6436 |
| NB5R3 | 0,8515 | 0,0321 | 0,0245 | 1,0379 | 0,6335 | 1,6384 |
| PSB2 | 0,9008 | 0,0035 | 0,0040 | 0,9808 | 1,6271 | 0,6028 |
| SPTA1 | 0,9570 | 0,0117 | 0,0126 | 1,0052 | 0,7695 | 1,3062 |
| SPTB1 | 0,9825 | 0,0005 | 0,0006 | 1,0029 | 0,5985 | 1,6759 |

Particularly, if we consider a fold change > 1.5 and a statistically significant p-value (< 0.05) (see **Table 1),** proteins LAMP2, GNAQ, LKHA4, HBD and/or SODM are particularly suitable to differentiate MBC from CT; proteins LAMP2, SODM, CISY, PSB2 and/or AP1B1 are particularly suitable to differentiate NMBC from CT; and proteins GNAQ, SPTB1, NB5R3 and/or NTF2 are particularly suitable to differentiate between NMBC and MBC.

Although any of the proteins included in **Table 1,** particularly any of the proteins having a fold change > 1.5 and a statistically significant p-value (< 0.05), or any combination thereof, can be used in the context of the present invention, protein Lysosome-associated membrane protein 2 (LAMP2) is considered as a particularly relevant protein since it offers a reduced p-value for differentiating both MBC and NMBC from CT associated with a fold change > 1.5.

So, the first embodiment of the present invention refers to an *in vitro* method (hereinafter method of the invention) for the diagnosis and/or prognosis of breast cancer in a patient which comprises: a) Measuring the concentration level of at least protein LAMP2 in RBCs comprised in blood samples isolated from the patient (i.e. RBCs present in- or obtained from an isolated patient blood sample); b) wherein if a deviation or variation in the concentration level is determined as compared with the concentration level determined in control subjects, this is indicative that the subject is suffering from breast cancer or that the subject has a bad prognosis, wherein bad prognosis means that the patient may suffer from MBC.

The second embodiment of the present invention refers to the *in vitro* use of LAMP2 in RBCs comprised in blood samples isolated from the patient for the diagnosis and/or prognosis of breast cancer.

In a preferred embodiment, the identification of a higher concentration level of protein LAMP2 as compared with a pre-established threshold value, is indicative that the subject is suffering from breast cancer or that the subject has a bad prognosis, wherein bad prognosis means that the patient may suffer from MBC.

In a preferred embodiment, the breast cancer is MBC or NMBC.

In a preferred embodiment, the level of the protein LAMP2 is measured in combination with cell distribution width (RDW) and hematocrit.

In a preferred embodiment, the level of the protein LAMP2 is measured in combination with the level of any of the proteins included in **Table 1.**

In a preferred embodiment, RBCs are analyzed by ELISA or lateral flow.

In a preferred embodiment the results obtained by implementing the method of the invention is confirmed by an image technique, for example by positron emission tomography (PET) or computed tomography (CT).

The third embodiment refers to a kit adapted for the diagnosis and/or prognosis of breast cancer in a patient which comprises tools or reagents for measuring the concentration level of LAMP2 in RBCs comprised in blood samples isolated from the patient (i.e. RBCs present in- or obtained from an isolated patient blood sample).

In a preferred embodiment, the kit comprises tools or reagents for measuring the concentration level of LAMP2 in RBCs comprised in blood samples isolated from the patient (i.e. RBCs present in- or obtained from an isolated patient blood sample) in combination with cell distribution width (RDW) and hematocrit.

In a preferred embodiment, the kit comprises tools or reagents for measuring the concentration level of LAMP2 in RBCs comprised in blood samples isolated from the patient (i.e. RBCs present in- or obtained from an isolated patient blood sample) in combination with the concentration level of any of the proteins included in **Table 1.**

In a preferred embodiment, the kit is ELISA or lateral flow.

The fourth embodiment of the present invention refer to the use the kit for the diagnosis and/or prognosis of breast cancer.

Such as it is indicated above, the present invention also includes the possibility of carrying out the above cited method by determining the concentration level of protein signatures comprising at least two of the proteins included in **Table 1.** Thus, according to the method of the invention, after measuring the concentration level of any possible combination of the proteins included in **Table 1,** a score value is obtained for the signature and this score value is compared with a threshold value which defines the diagnostic rule. If this score value is higher/lower than the threshold, then the corresponding sample is classified as a positive sample, which is an indication that the patient might be suffering from breast cancer. The threshold value has been defined in order to optimize sensitivity and specificity values. Consequently, in a preferred embodiment, the method of the invention comprises: a) Measuring the concentration level of any of the above cited combinations of biomarkers, in a biological sample obtained from the subject, b) processing the concentration values in order to obtain a risk score and c) wherein if a deviation or variation of the risk score value obtained for any of the above cited combinations of biomarkers is identified, as compared with a reference value, this is indicative that the subject is suffering from breast cancer.

The fifth embodiment of the present invention refers to a method for treating a patient suffering from breast cancer (metastatic or non-metastatic breast cancer) which comprises a first step wherein the patients is diagnosed or classified using any of the methods or kits described above. In a preferred embodiment the patient is treated for example by surgery, hormone therapy, chemotherapy, radiotherapy, anti-Her2 or cyclin inhibitors, once she has been diagnosed following the method of the invention.

The sixth embodiment of the present invention refers to a method for analyzing a blood sample isolated from the patient, prior to diagnosis of breast cancer, for determining the concentration level of any of the proteins included in **Table 1** in RBCs, wherein an increase in the concentration level of any of the proteins included in **Table 1** in RBCs indicates that the blood sample may contain RBCs derived from breast cancer patients. RBCs derived from breast cancer patients tend to form rosettes and adhere to the CTCs and to cancer cell lines *in vitro.*

The seventh embodiment of the present invention refers to a computer-implemented invention, wherein a processing unit (hardware) and a software are configured to:
- Receive the concentration level values of any of the proteins included in **Table 1,**
- Process the concentration level values received for finding substantial variations or deviations, and
- Provide an output through a terminal display of the variation or deviation of the concentration level, wherein the variation or deviation of the concentration level indicates that the subject may be suffering from breast cancer.

The eight embodiment of the present invention refers to a method for detecting any of the proteins included in **Table 1** in RBCs comprised in blood samples from a subject at risk of developing breast cancer, preferably a subject at risk of developing MBC, the method comprising: i) Isolating RBCs from the blood samples and ii) determining the concentration of the proteins, for example by performing an immunoassay or lateral flow.

The last embodiment of the present invention refers to a method for the diagnosis or prognosis of breast cancer, preferably MBC in a patient, which comprises determining that the patient has a risk of developing breast cancer based on a fold change of at least 1.5 of any of the proteins included in **Table 1** when compared to healthy control.

For the purpose of the present invention:
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- The concentration "reference concentration level measured in healthy control subjects", refer to a "reference value" of the concentration level of the proteins. If a deviation of the concentration level of the proteins is determined with respect to said "reference concentration level measured in healthy control subjects", this is an indication of breast cancer. Particularly, if the concentration level of the biomarkers or signatures of the present invention are significantly higher or lower with respect to said "reference value" this is an indication of breast cancer.
- The concentration "risk score" refers to a risk value obtained after processing one or more concentration values into a single value (or risk value), which represents the probability of disease for the individual. This risk value will be compared with a reference value to evaluate if the patient might be suffering from breast cancer.
- A "reference value" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare the biomarker levels (or scores) obtained according to the method of the invention with a defined threshold value. Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0.

### Description of the figures

**Figure 1****. A)** PBMCs after Ficoll density gradient protocol from a healthy donor (H) and metastatic Breast Cancer patient (P). **B)** pre-enriched CTCs from breast cancer patient in culture. RBCs is forming a matrix (Rossete) among them and with tumour cells. **C)** RDW correlation with RBCs matrix formation in culture. **D)** Incubation of RBCs from breast cancer patient with MDA-MB-231-GFP BC cell lines (breast cancer cell). **E)** Incubation of RBCs from breast cancer patient with MCF7 BC cell lines (breast cancer cell). **F)** Incubation of RBCs from healthy donor with MCF7 BC cell lines (breast cancer cell).

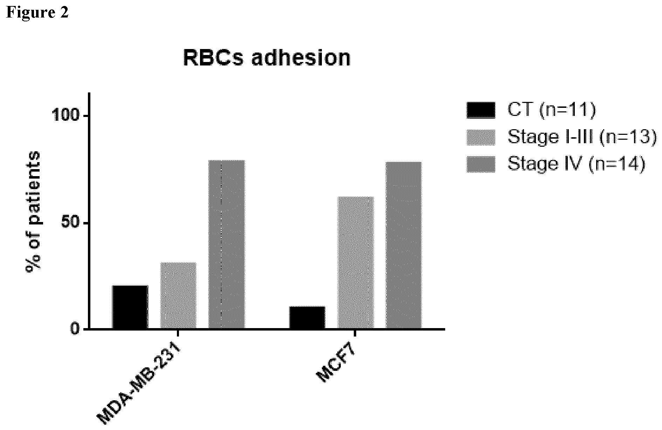

**Figure 2****.** Percentage of patients that shown RBCs bound to breast tumor cell line (MCF7 or MDA-MB-231) depending on the stage (Stage I-III in clear grey) and stage IV (dark grey) and healthy donors (black).

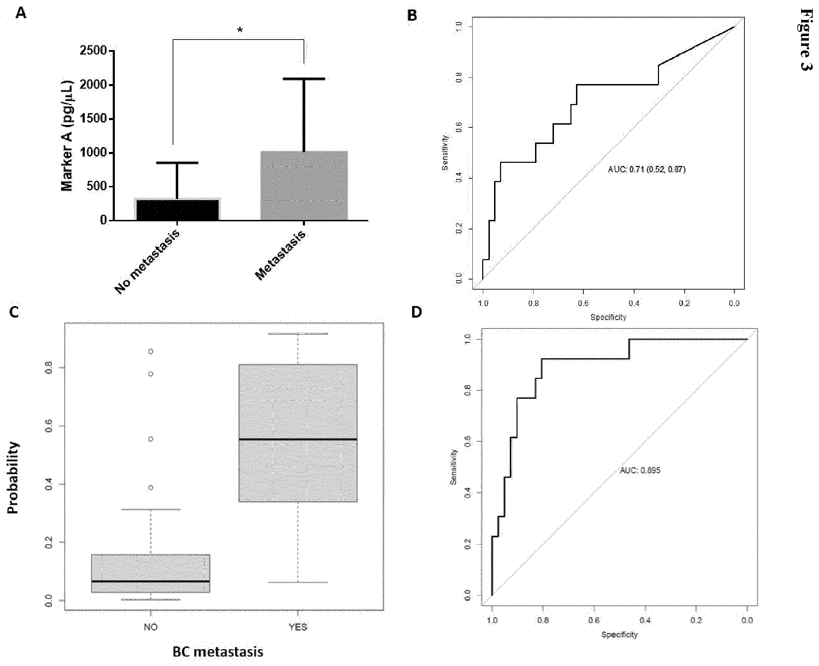

**Figure 3****. A)** ELISA data (pg/µL) of LAMP2 in metastatic (n=16) and non-metastatic (localized BC and controls) (n=44) women. Non-metastatic patients were recruited pre-surgery. Controls samples were obtained from women with paired age to the patients. **B)** ROC Curve for LAMP2. The AUC is 0,71. **C)** Probability of being metastatic given by the logistic model. **D)** ROC Curve for the model using LAMP2+RDW+Hematocrit. AUC=0,89.

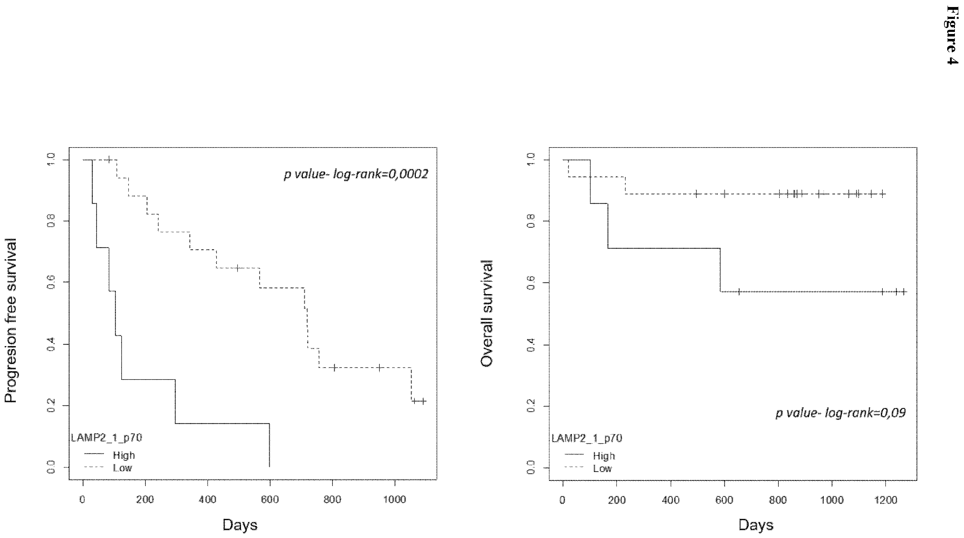

**Figure 4****.** Kaplan-Meier plots for PFS and OS for LAMP2 levels in MBC patients: high (black) and low (red). The threshold to define the levels were percentile 70 of the values from SWATH analysis. P-values were calculated using the log-rank test.

### Detailed description of the invention

### Examples

### Example 1. Proteomic analysis.

25 NMBC patients, 27 MBC patient (including all BC molecular subtypes) and 33 CT healthy donors blood samples were collected, and RBCs membrane proteins were extracted. Proteomic analysis was performed using SWATH technology. We found a different proteomic profile among patients and healthy donors **(Table 1).** Furthermore, we had seen that in MBC patients, RDW is altered in 50% of analyzed patients (n=30) and that the number of CTCs correlates with the alteration of RDW value. **Table 1** shows the differential proteins expressed in controls, non metastatic Breast Cancer Patients (NMBC) and metastatic Breast Cancer Patients (MBC), the p-value of the t-test analysis and fold change.

### Example 2. RBCs adhere to CTCs in MBC (metastatic breast cancer cells).

We found some interesting evidences which suggest that RBCs adhere to CTCs in mBC:
I. In most of the metastatic patients analyzed, we found a not negligible number of RBCs in the CTCs fraction (after CTCs pre-enrichment protocol). Moreover, when isolating peripheral blood mononuclear cells (PBMCs) using Ficoll density gradient centrifugation protocol, RBCs were present in the white fraction, a phenomenon not seen in non-advanced cancer patients or healthy donors **(****Figure 1A****).** It is noteworthy that the amount of "contaminant" RBCs is proportional to the stage of the disease. We have seen that the presence of RBCs accompanying CTCs isolated from the blood of metastatic patients predicts PFS and OS.
II. In more than 80% of the analyzed samples (n=33), pre-enriched CTCs form an odd network with RBCs in vitro (unspecific isolated with CTCs) **(****Figure 1B**)**.** Moreover, this behavior is associated with high RDW value in the analyzed samples **(****Figure 1C****).**
III. *In vitro* incubation of RBC from mBC patients with BC cell lines leads to the formation of RBC-tumor cell bonds **(****Figure 1D, E****),** not seen in healthy donors **(****Figure 1F**). We performed an in vitro incubation of RBC from metastatic breast cancer patients and RBCs from healthy donors with two different breast cancer cell lines (MCF7 and MD-MB-231). The percentage of patients whose RBCs adhere to tumor cell line is shown in **Figure 2****.**

### Example 4. Elisa assay

In a different cohort of patients, we analysed the protein concentration of LAMP2, using an ELISA assay (sandwich type) in 60 samples (Metastatic, n=16; non-metastatic, n=24 and controls, n=20), 41 out of 60 samples were new. The results confirmed that LAMP2 concentration (pg/L) was higher in the metastatic group than in non-metastatic or control individuals (p-value = 0.008639) **(****Figure 3A****).**

Although, LAMP2 is present in other cells of the white cell fraction of the blood, it is not used in clinics and has not been identified in RBCs so far. Additionally, we analysed the concentration of LAMP2 in plasma of BC patients and controls by ELISA. We did not find differences in the concentration levels of this marker between both groups. Since LAMP2 is present in other blood cells, the lysis of these cells could be shedding this protein to the plasma. Thus, we discard the use of plasma instead of RBCs as did not show specificity for metastatic patients.

### Example 5. Statistical analysis. ROC curve and logistic model.

We performed a Receiver operating characteristic curve (ROC) analysis for the assessment of the diagnostic accuracy of LAMP2. The AUC (area under the curve) was 0.71 with 76% of sensitivity and a specificity of 62 % **(****Figure 2B****).** Thus, having higher levels of LAMP2 (based on the best threshold given by the model) would be indicative of metastatic disease. Next, we developed a Logistic regression ROC model combining LAMP2, RDW and hematocrit levels for mBC patients' discrimination. RDW and hematocrit levels are routinely analysed in blood tests. This model has an AUC of 0.89 in the ROC curve. The accuracy of the model is 92.3 % of sensitivity, 80.5 % of specificity and a total success rate of 83.33% **(****Figure 2 C-D****).**

## Claims

1. *In vitro* method for the diagnosis and/or prognosis of breast cancer in a patient which comprises: a) Measuring the concentration level of at least protein LAMP2 in red blood cells (RBCs) comprised in blood samples isolated from the patient; b) wherein if a statistically higher concentration level is determined as compared with a pre-established threshold value, this is indicative that the subject is suffering from breast cancer or that the subject has a bad prognosis, wherein bad prognosis means that the patient may suffer from metastatic breast cancer.

2. *In vitro* method, according to claim 1, wherein the breast cancer diagnosed in the patient is metastatic or non-metastatic breast cancer.

3. *In vitro* method, according to any of the previous claims, wherein the level of the protein LAMP2 is measured in combination with cell distribution width (RDW) and hematocrit.

4. *In vitro* method, according to any of the previous claims, which further comprises measuring the level of any of the proteins included in Table 1.

5. *In vitro* method, according to any of the previous claims, wherein the RBCs are analyzed by ELISA or lateral flow.

6. *In vitro* use of LAMP2 present in RBCs comprised in blood samples isolated from the patient for the diagnosis and/or prognosis of breast cancer.

7. *In vitro* use, according to claim 6, wherein the breast cancer is metastatic or non-metastatic breast cancer.

8. *In vitro* use, according to any of the claims 6 or 7, in combination with cell distribution width (RDW) and hematocrit.

9. *In vitro* use, according to any of the claims 6 to 8, in combination with any of the proteins included in Table 1.

10. Kit adapted for the diagnosis and/or prognosis of breast cancer in a patient which comprises tools or reagents for measuring the concentration level of LAMP2 in RBCs comprised in blood samples isolated from the patient.

11. Kit, according to claim 10, adapted for the diagnosis and/or prognosis of breast cancer in a patient which comprises tools or reagents for measuring the concentration level of LAMP2 in RBCs comprised in blood samples obtained from the patient in combination with cell distribution width (RDW) and hematocrit.

12. Kit, according to any of the claims 10 or 11, adapted for the diagnosis and/or prognosis of breast cancer in a patient which comprises tools or reagents for measuring the concentration level of LAMP2 in RBCs comprised in blood samples obtained from the patient in combination with the concentration level of any of the proteins included in Table 1.

13. Kit, according to any of the claims 10 to 12, **characterized in that** it is an ELISA or lateral flow.

14. Use of the kit according to any of the claims 10 to 13 for the diagnosis and/or prognosis of breast cancer.
